# EUROPEAN PATENT APPLICATION

(11) **EP 4 652 994 A1**
(43) Date of publication of application: **26.11.2025**
(21) Application number: 23917842.9
(22) Date of filing: 22.08.2023
(51) Int. Cl.: A61K 31/415, A61P 17/00, A61P 29/00, A23L 33/10

(54) **COMPOSITION FOR PREVENTING OR TREATING INFLAMMATORY SKIN DISEASES, CONTAINING 1H-PYRAZOLE-3-AMIDE COMPOUND DERIVATIVE**

(30) Priority: 19.01.2023 KR 20230007871
(71) Applicant: Ajou University Industry-Academic Cooperation Foundation, Suwon-si, Gyeonggi-do 16499 (KR)
(72) Inventor: KIM, Wook, Suwon-si Gyeonggi-do 16509 (KR); KIM, Eunha, Seongbuk-gu Seoul 02794 (KR); HAN, Ji Hye, Suwon-si Gyeonggi-do 16689 (KR); CHOI, Hong-Seo, Gunpo-si Gyeonggi-do 15875 (KR); KIM, Eun-Su, Hwaseong-si Gyeonggi-do 18600 (KR); KIM, Suha, Yuseong-gu Daejeon 34061 (KR)
(74) Representative: Mewburn Ellis LLP
(86) International application number: PCT/KR2023/012410
(87) International publication number: WO 2024/154884

(57) **Abstract**

The present invention relates to a composition for preventing or treating inflammatory skin diseases, containing a 1H-pyrazole-3-amide compound derivative. Found to reduce TNF-α, IL-1β, IL-17A, and Ki67 in the skin of mouse models of psoriasis or contact dermatitis, the composition can prevent or treat such inflammatory skin diseases by effectively inhibiting psoriasis or contact dermatitis.

## Description

### Technical Field

The present disclosure provides a composition for preventing or treating an inflammatory skin disease, including a 1H-pyrazole-3-amide compound derivative.

### Background Art

Psoriasis is an intractable autoimmune skin disease accompanied by hyperproliferation of epidermis layers as well as erythema and ulcers of various sizes. The mechanism of psoriasis is mainly involved with the hyperproliferation and hyperactivation of keratinocytes, which is induced by Th17 cell responses. Looking at the process, production of TNF-α and IL-23 due to overactivation of initial dendritic cells in skin maximizes inflammatory effects of Th17 cells, resulting in an increase in the production of IL-17 to lead to proliferation of keratinocytes. Additionally, keratinocytes produce additional inflammatory cytokines and chemokines in response to stimulation such as TNF-α, thereby promoting and perpetuating tissue infiltration by inflammatory leukocytes. Recently, antibody treatments that block the IL-23-IL-17 pathway have been relatively effective, but the effectiveness is temporary and weak in patients with high severity.

Contact dermatitis is an eczematous skin disease occurring in the area of contact as an allergen repeatedly penetrates the skin. The onset of this type of contact dermatitis is a cell-mediated hypersensitivity caused by T lymphocytes and macrophages, which is called delayed type hypersensitivity since the inflammatory reaction takes place in a relatively late stage, within a few hours after contact with the antigen. In recent years, with the development of material civilization, the incidence of contact dermatitis is increasing compared to the past due to environmental pollution as well as mass production of new substances with diversity in types. Everyone experiences contact dermatitis at least a few times throughout their lives, and the causes thereof also vary to cause contact dermatitis by natural substances, industrial products, and even medications.

There is a need for development of new and effective drugs with new mechanisms of action for the psoriasis or contact dermatitis.

### Disclosure of the Invention

### Technical Goals

An object of the present disclosure is to provide a pharmaceutical composition capable of effectively preventing and treating an inflammatory skin disease.

Another object of the present disclosure is to provide a health functional food composition capable of effectively preventing and ameliorating an inflammatory skin disease. Technical Solutions

To achieve the above objects, the present disclosure provides a pharmaceutical composition for preventing or treating an inflammatory skin disease, including a 1H-pyrazole-3-amide compound represented by the following Chemical Formula 1 or a pharmaceutically acceptable salt thereof as an active ingredient.

In addition, the present disclosure provides a health functional food composition for preventing or ameliorating an inflammatory skin disease, including a 1H-pyrazole-3-amide compound represented by the following Chemical Formula 1 or a pharmaceutically acceptable salt thereof as an active ingredient.

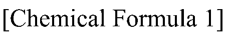

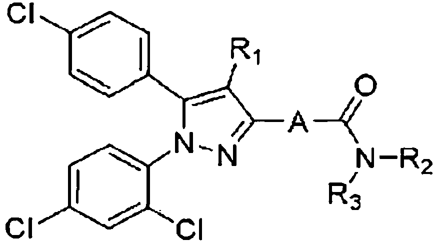

In the Chemical Formula 1, A is 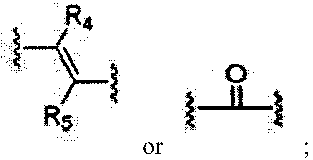 R₁ is hydrogen, (C1-C10)alkyl, (C3-C7)cycloalkyl, (C6-C20)aryl, or (C6-C20)ar(C1-C10)alkyl; R₂ and R₃ are independently hydrogen, (C1-C10)alkyl, (C6-C20)aryl, (C6-C20)ar(C1-C10)alkyl, (C3-C7)cycloalkyl, (C3-C7)heterocycloalkyl including one or more heteroatoms selected from N, O, and S, adamantyl, piperidino, or pyrrolidino or R₂ and R₃ may be connected to (C3-C7)alkylene to form a ring, and one or more carbon atoms in the alkylene may be substituted with NR₆, O, or S; R₄ and R₅ are each independently hydrogen, (C1-C10)alkyl, (C3-C7)cycloalkyl, or (C6-C20)aryl; R₆ is hydrogen, (C1-C10)alkyl, or (C1-C10)alkoxycarbonyl; and the alkyl, aryl, aralkyl, cycloalkyl, adamantly, or heterocycloalkyl in the R₁, R₂, and R₃ may be further substituted with one or more substituents selected from halogen, (C1-C10)alkyl, halo(C1-C10)alkyl, (C1-C10)alkoxy, mono or di-(C1-C10)alkylamino, mono or di-(C6-C20)arylamino, (C3-C7)cycloalkyl, (C2-C20)heteroaryl, hydroxy, (C3-C7)heterocycloalkyl including one or more heteroatoms selected from N, O, and S, pyrrolidino, or piperidino.

### Advantageous Effects

The present disclosure relates to a composition for preventing or treating an inflammatory skin disease including a 1H-pyrazole-3-amide compound derivative, wherein it was found that TNF-α, IL-1β, IL-17A, and Ki67 are reduced in the skin of mouse models with psoriasis or contact dermatitis, such that the composition is capable of preventing or treating such inflammatory skin diseases by effectively suppressing psoriasis or contact dermatitis.

### Brief Description of Drawings

FIG. 1 shows structural formulas of 1H-pyrazole-3-amide compounds used in one embodiment of the present disclosure.
FIG. 2 shows results of identifying a PASI score and spleen weight in a psoriasis mouse model.
FIG. 3 shows results of identifying a skin thickness in a psoriasis mouse model.
FIG. 4 shows results of identifying a level of TNF-α expression in a psoriasis mouse model.
FIG. 5 shows results of identifying a level of IL-1β expression in a psoriasis mouse model.
FIG. 6 shows identification of a therapeutic effect on dermatitis in a contact dermatitis mouse model.
FIG. 7 shows results of identifying a skin thickness in a contact dermatitis mouse model.
FIG. 8 shows results of identifying a level of IL-1β expression in a contact dermatitis mouse model.
FIG. 9 shows results of identifying a level of IL-17A expression in a contact dermatitis mouse model.

### Best Mode for Carrying Out the Invention

Hereinafter, the present disclosure will be described in more detail.

The present disclosure provides a pharmaceutical composition for preventing or treating an inflammatory skin disease, including a 1H-pyrazole-3-amide compound represented by the following Chemical Formula 1 or a pharmaceutically acceptable salt thereof as an active ingredient;

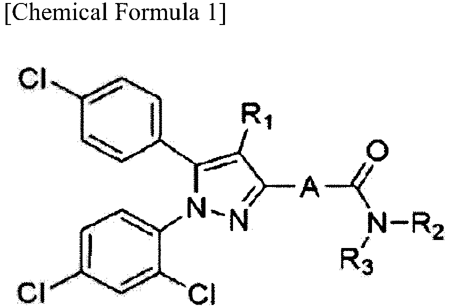

wherein, in the Chemical Formula 1, A is 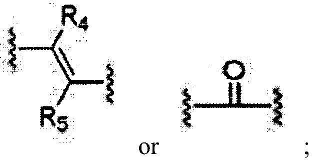 R₁ is hydrogen, (C1-C10)alkyl, (C3-C7)cycloalkyl, (C6-C20)aryl, or (C6-C20)ar(C1-C10)alkyl; R₂ and R₃ are independently hydrogen, (C1-C10)alkyl, (C6-C20)aryl, (C6-C20)ar(C1-C10)alkyl, (C3-C7)cycloalkyl, (C3-C7)heterocycloalkyl including one or more heteroatoms selected from N, O, and S, adamantyl, piperidino, or pyrrolidino or R₂ and R₃ may be connected to (C3-C7)alkylene to form a ring, and one or more carbon atoms in the alkylene may be substituted with NR₆, O, or S; R₄ and R₅ are each independently hydrogen, (C1-C10)alkyl, (C3-C7)cycloalkyl, or (C6-C20)aryl; R₆ is hydrogen, (C1-C10)alkyl, or (C1-C10)alkoxycarbonyl; and the alkyl, aryl, aralkyl, cycloalkyl, adamantly, or heterocycloalkyl in the R₁, R₂, and R₃ may be further substituted with one or more substituents selected from halogen, (C1-C10)alkyl, halo(C1-C10)alkyl, (C1-C10)alkoxy, mono or di-(C1-C10)alkylamino, mono or di-(C6-C20)arylamino, (C3-C7)cycloalkyl, (C2-C20)heteroaryl, hydroxy, (C3-C7)heterocycloalkyl including one or more heteroatoms selected from N, O, and S, pyrrolidino, or piperidino.

Specifically, in the Chemical Formula 1, R₁ is (C1-C10)alkyl, R₂ and R₃ are each independently hydrogen, (C1-C10)alkyl, (C6-C20)aryl, (C6-C20)ar(C1-C10)alkyl, (C3-C7)cycloalkyl, piperidino, adamantyl, or piperidinyl or R₂ and R₃ may be connected to (C4-C6)alkylene to form a ring, and one or more carbon atoms in the alkylene may be substituted with NR₆, O, or S; R₄ and R₅ are hydrogen; R₆ is hydrogen, (C1-C10)alkyl, or (C1-C10)alkoxycarbonyl; and the alkyl, aryl, or aralkyl in the R₂ and R₃ may be further substituted with one or more substituents selected from halogen, (C1-C10)alkyl, halo(C1-C10)alkyl, (C1-C10)alkoxy, mono or di-(C1-C10)alkylamino, (C3-C7)cycloalkyl, (C2-C20)heteroaryl, or hydroxy.

Preferably, the 1H-pyrazol-3-amide compound may be selected from the group consisting of (E)-N-(4-chlorophenyl)-3-(5-(4-chlorophenyl)- 1 -(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-phenylacrylamide; (E)-N-(3-chlorobenzyl)-3-(5-(4-chlorophenyl)-1-(2,4-dichlorophenyl)-4-methyl-1H-pyrazol-3-yl)acrylamide; (E)-N-(2-chlorobenzyl)-3-(5-(4-chlorophenyl)-1-(2,4-dichlorophenyl)-4-methyl-1H-pyrazol-3-yl)acrylamide; (E)-N-benzyl-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(4-methoxybenzyl)acrylamide; (E)-N-(4-chlorobenzyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(4-fluorobenzyl)acrylamide; (E)-3-(5-(4-chlorophenyl)-1-(2,4-dichlorophenyl)-4-methyl-1H-pyrazol-3-yl)-N-(3-fluorobenzyl)acrylamide; (E)-3-(5-(4-chlorophenyl)-1-(2,4-dichlorophenyl)-4-methyl-1H-pyrazol-3-yl)-N-isopropylacrylamide; (E)-3-(5-(4-chlorophenyl)-1-(2,4-dichlorophenyl)-4-methyl-1H-pyrazol-3-yl)-N-(3-chloropropyl)acrylamide; (E)-3-(5-(4-chlorophenyl)-1-(2,4-dichlorophenyl)-4-methyl-1H-pyrazol-3-yl)-N-hexylacrylamide; (E)-3-(5-(4-chlorophenyl)-1-(2,4-dichlorophenyl)-4-methyl-1H-pyrazol-3-yl)-N-(cyclohexylmethyl)acrylamide; (E)-3-(5-(4-chlorophenyl)-1-(2,4-dichlorophenyl)-4-methyl-1H-pyrazol-3-yl)-N-(3-(trifluoromethyl)benzyl)acrylamide; (E)-3-(5-(4-chlorophenyl)-1-(2,4-dichlorophenyl)-4-methyl-1H-pyrazol-3-yl)-N-(4-trifluoromethyl)benzyl)acrylamide; (E)-3-(5-(4-chlorophenyl)-1-(2,4-dichlorophenyl)-4-methyl-1H-pyrazol-3-yl)-N-(4-(dimethylamino)benzyl)acrylamide; (E)-N-(3-chlorophenethyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(piperidin-1-yl)acrylamide; (E)-3-(5-(4-chlorophenyl)-1-(2,4-dichlorophenyl)-4-methyl-1H-pyrazol-3-yl)-N-(furan-3-ylmethyl)acrylamide; (E)-4-(5-(4-chlorophenyl)-1-(2,4-dichlorophenyl)-4-methyl-1H-pyrazol-3-yl)-1-(piperidin-4-yl)acrylamide; 2-(5-(4-chlorophenyl)-1-(2,4-dichlorophenyl)-4-methyl-1H-pyrazol-3-yl)-2-oxo-N-phenylacetamide; 2-(5-(4-chlorophenyl)-1-(2,4-dichlorophenyl)-4-methyl-1H-pyrazol-3-yl)-N-methyl-2-oxo-N-phenylacetamide; N-(3-chlorobenzyl)-2-(5-(4-chlorophenyl)-1-(2,4-dichlorophenyl)-4-methyl-1H-pyrazol-3-yl)-2-oxoacetamide; N-(3-chlorophenethyl)-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-N-cyclopentyl-2-oxoacetamide; 2-(5-(4-chlorophenyl)-1-(2,4-dichlorophenyl)-4-methyl-1H-pyrazol-3-yl)-N-cyclohexyl-2-oxoacetamide; 2-(5-(4-chlorophenyl)-1-(2,4-dichlorophenyl)-4-methyl-1H-pyrazol-3-yl)-N-cycloheptyl-2-oxoacetamide; 1-(5-(4-chlorophenyl)-1-(2,4-dichlorophenyl)-4-methyl-1H-pyrazol-3-yl)-2-(pyrrolidin-1-yl)ethane-1,2-dione; 1-(5-(4-chlorophenyl)-1-(2,4-dichlorophenyl)-4-methyl-1H-pyrazol-3-yl)-2-(piperidin-1-yl)ethane-1,2-dione; 1-(azepan-1-yl)-2-(5-(4-chlorophenyl)-1-(2,4-dichlorophenyl)-4-methyl-1H-pyrazol-3-yl)ethane-1,2-dione; 2-(5-(4-chlorophenyl)-1-(2,4-dichlorophenyl)-4-methyl-1H-pyrazol-3-yl)-2-oxo-N-(piperidin-1-yl)acetamide; 2-(5-(4-chlorophenyl)-1-(2,4-dichlorophenyl)-4-methyl-1H-pyrazol-3-yl)-N-(cyclohexylmethyl)-2-oxoacetamide; 1-(5-(4-chlorophenyl)-1-(2,4-dichlorophenyl)-4-methyl-1H-pyrazol-3-yl)-2-(piperazin-1-yl)ethane-1,2-dione; tert-butyl 4-(2-(5-(4-chlorophenyl)-1-(2,4-dichlorophenyl)-4-methyl-1H-pyrazol-3-yl)-2-oxoacetyl)piperazine-1-carboxylate; 1-(5-(4-chlorophenyl)-1-(2,4-dichlorophenyl)-4-methyl-1H-pyrazol-3-yl)-2-(4-methylpiperazin-1-yl)ethane-1,2-dione; 1-(5-(4-chlorophenyl)-1-(2,4-dichlorophenyl)-4-methyl-1H-pyrazol-3-yl)-2-morpholinoethane-1,2-dione; 1-(5-(4-chlorophenyl)-1-(2,4-dichlorophenyl)-4-methyl-1H-pyrazol-3-yl)-2-thiomorpholinoethane-1,2-dione; 2-(5-(4-chlorophenyl)-1-(2,4-dichlorophenyl)-4-methyl-1H-pyrazol-3-yl)-N-adamantyl-2-oxoacetamide; 2-(5-(4-chlorophenyl)-1-(2,4-dichlorophenyl)-4-methyl-1H-pyrazol-3-yl)-N,N-dicyclohexyl-2-oxoacetamide; 2-(5-(4-chlorophenyl)-1-(2,4-dichlorophenyl)-4-methyl-1H-pyrazol-3-yl)-N,N-dihexyl-2-oxoacetamide; 2-(5-(4-chlorophenyl)-1-(2,4-dichlorophenyl)-4-methyl-1H-pyrazol-3-yl)-N-ethyl-2-oxoacetamide; 2-(5-(4-chlorophenyl)-1-(2,4-dichlorophenyl)-4-methyl-1H-pyrazol-3-yl)-2-oxo-N-propylacetamide; 2-(5-(4-chlorophenyl)-1-(2,4-dichlorophenyl)-4-methyl-1H-pyrazol-3-yl)-N-isopropyl-2-oxoacetamide; N-butyl-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-N-isobutyl-2-oxoacetamide; N-tert-butyl-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-2-oxo-N-pentylacetamide; 2-(5-(4-chlorophenyl)-1-(2,4-dichlorophenyl)-4-methyl-1H-pyrazol-3-yl)-N-hexyl-2-oxoacetamide; 2-(5-(4-chlorophenyl)-1-(2,4-dichlorophenyl)-4-methyl-1H-pyrazol-3-yl)-N-heptyl-2-oxoacetamide; 2-(5-(4-chlorophenyl)-1-(2,4-dichlorophenyl)-4-methyl-1H-pyrazol-3-yl)-N-octyl-2-oxoacetamide; and 2-(5-(4-chlorophenyl)-1-(2,4-dichlorophenyl)-4-methyl-1H-pyrazol-3-yl)-N-(2-hydroxyethyl)-2-oxoacetamide, but is not limited thereto.

More preferably, the 1H-pyrazole-3-amide compound may be (E)-N-(3-chlorobenzyl)-3-(5-(4-chlorophenyl)-1-(2,4-dichlorophenyl)-4-methyl-1H-pyrazol-3-yl)acrylamide or 2-(5-(4-chlorophenyl)-1-(2,4-dichlorophenyl)-4-methyl-1H-pyrazol-3-yl)-N-(2-hydroxyethyl)-2-oxoacetamide.

The inflammatory skin disease may be selected from the group consisting of atopic dermatitis, allergic dermatitis, psoriasis, seborrheic dermatitis, contact dermatitis, lupus erythematosus, and papular urticaria, but is not limited thereto.

In another embodiment of the present disclosure, the pharmaceutical composition may further include one or more additives selected from the group consisting of appropriate carriers, excipients, disintegrators, sweeteners, coating agents, leavening agents, lubricants, glydents, flavoring agents, antioxidants, buffers, bacteriostatic agents, diluents, dispersants, surfactants, binders, and lubricants that are commonly used in the manufacture of pharmaceutical compositions. Specifically, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearate, and mineral oil may be used as the carriers, excipients, and diluents, solid preparations for oral administration may include tablets, pills, acids, granules, and capsules, and these solid preparations may be prepared by mixing, in the composition, at least one or more excipients, for example, starch, calcium carbonate, sucrose or lactose, and gelatin. In addition to simple excipients, lubricants such as magnesium stearate and talc may also be used. Oral liquid preparations may include suspensions, liquids, emulsions, and syrups, and various excipients, such as humectants, sweeteners, fragrances, and preservatives may be included in addition to the simple diluents that are commonly used, such as water and liquid paraffin. Preparations for parenteral administration include sterile aqueous solutions, non-aqueous solvents, suspensions, emulsions, lyophilized agents, and suppositories. For non-aqueous solvents and suspensions, propylene glycol, polyethylene glycol, vegetable oils such as olive oil, and injectable esters such as ethyl oleate may be used. As for base materials of suppositories, witepsol, macrogol, tween 61, cacao butter, laurin fat, and glycerogelatin may be used. According to one embodiment of the present disclosure, the pharmaceutical composition may be administered to a subject in a conventional manner through intravenous, intraarterial, intraperitoneal, intramuscular, intraarterial, intraperitoneal, intrasternal, transdermal, intranasal, inhalation, topical, rectal, oral, intraocular, or intradermal routes. The dosage of the active ingredient according to the present disclosure may vary depending on the condition and body weight of the subject, the type and severity of the disease, the form of the drug, and the route and duration of administration and may be appropriately selected by those skilled in the art, and the daily dose may be 0.01 mg/kg to 200 mg/kg, preferably 0.1 mg/kg to 200 mg/kg, and more preferably 0.1 mg/kg to 100 mg/kg. The administration may be conducted once a day or divided into several doses, but the scope of the present disclosure is not limited thereby.

In addition, the present disclosure provides a health functional food composition for preventing or ameliorating an inflammatory skin disease, including a 1H-pyrazole-3-amide compound represented by the following Chemical Formula 1 or a pharmaceutically acceptable salt thereof as an active ingredient;

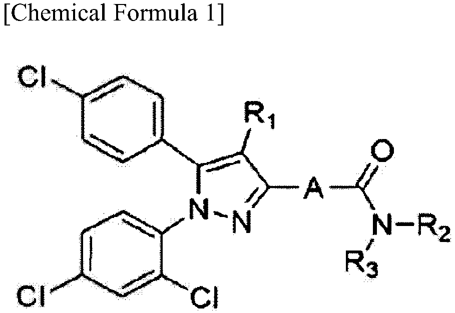

wherein, in the Chemical Formula 1, A is 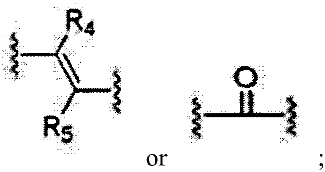 R₁ is hydrogen, (C1-C10)alkyl, (C3-C7)cycloalkyl, (C6-C20)aryl, or (C6-C20)ar(C1-C10)alkyl; R₂ and R₃ are independently hydrogen, (C1-C10)alkyl, (C6-C20)aryl, (C6-C20)ar(C1-C10)alkyl, (C3-C7)cycloalkyl, (C3-C7)heterocycloalkyl including one or more heteroatoms selected from N, O, and S, adamantyl, piperidino, or pyrrolidino or R₂ and R₃ may be connected to (C3-C7)alkylene to form a ring, and one or more carbon atoms in the alkylene may be substituted with NR₆, O, or S; R₄ and R₅ are each independently hydrogen, (C1-C10)alkyl, (C3-C7)cycloalkyl, or (C6-C20)aryl; R₆ is hydrogen, (C1-C10)alkyl, or (C1-C10)alkoxycarbonyl; and the alkyl, aryl, aralkyl, cycloalkyl, adamantly, or heterocycloalkyl in the R₁, R₂, and R₃ may be further substituted with one or more substituents selected from halogen, (C1-C10)alkyl, halo(C1-C10)alkyl, (C1-C10)alkoxy, mono or di-(C1-C10)alkylamino, mono or di-(C6-C20)arylamino, (C3-C7)cycloalkyl, (C2-C20)heteroaryl, hydroxy, (C3-C7)heterocycloalkyl including one or more heteroatoms selected from N, O, and S, pyrrolidino, or piperidino.

In the Chemical Formula 1, R₁ is (C1-C10)alkyl, R₂ and R₃ are each independently hydrogen, (C1-C10)alkyl, (C6-C20)aryl, (C6-C20)ar(C1-C10)alkyl, (C3-C7)cycloalkyl, piperidino, adamantyl, or piperidinyl or R₂ and R₃ may be connected to (C4-C6)alkylene to form a ring, and one or more carbon atoms in the alkylene may be substituted with NR₆, O, or S; R₄ and R₅ are hydrogen; R₆ is hydrogen, (C1-C10)alkyl, or (C1-C10)alkoxycarbonyl; and the alkyl, aryl, or aralkyl in the R₂ and R₃ may be further substituted with one or more substituents selected from halogen, (C1-C10)alkyl, halo(C1-C10)alkyl, (C1-C10)alkoxy, mono or di-(C1-C10)alkylamino, (C3-C7)cycloalkyl, (C2-C20)heteroaryl, or hydroxy.

The 1H-pyrazol-3-amide compound may be selected from the group consisting of (E)-N-(4-chlorophenyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-phenylacrylamide; (E)-N-(3-chlorobenzyl)-3-(5-(4-chlorophenyl)-1-(2,4-dichlorophenyl)-4-methyl-1H-pyrazol-3-yl)acrylamide; (E)-N-(2-chlorobenzyl)-3-(5-(4-chlorophenyl)-1-(2,4-dichlorophenyl)-4-methyl-1H-pyrazol-3-yl)acrylamide; (E)-N-benzyl-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(4-methoxybenzyl)acrylamide; (E)-N-(4-chlorobenzyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(4-fluorobenzyl)acrylamide; (E)-3-(5-(4-chlorophenyl)-1-(2,4-dichlorophenyl)-4-methyl-1H-pyrazol-3-yl)-N-(3-fluorobenzyl)acrylamide; (E)-3-(5-(4-chlorophenyl)-1-(2,4-dichlorophenyl)-4-methyl-1H-pyrazol-3-yl)-N-isopropylacrylamide; (E)-3-(5-(4-chlorophenyl)-1-(2,4-dichlorophenyl)-4-methyl-1H-pyrazol-3-yl)-N-(3-chloropropyl)acrylamide; (E)-3-(5-(4-chlorophenyl)-1-(2,4-dichlorophenyl)-4-methyl-1H-pyrazol-3-yl)-N-hexylacrylamide; (E)-3-(5-(4-chlorophenyl)-1-(2,4-dichlorophenyl)-4-methyl-1H-pyrazol-3-yl)-N-(cyclohexylmethyl)acrylamide; (E)-3-(5-(4-chlorophenyl)-1-(2,4-dichlorophenyl)-4-methyl-1H-pyrazol-3-yl)-N-(3-(trifluoromethyl)benzyl)acrylamide; (E)-3-(5-(4-chlorophenyl)-1-(2,4-dichlorophenyl)-4-methyl-1H-pyrazol-3-yl)-N-(4-trifluoromethyl)benzyl)acrylamide; (E)-3-(5-(4-chlorophenyl)-1-(2,4-dichlorophenyl)-4-methyl-1H-pyrazol-3-yl)-N-(4-(dimethylamino)benzyl)acrylamide; (E)-N-(3-chlorophenethyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(piperidin-1-yl)acrylamide; (E)-3-(5-(4-chlorophenyl)-1-(2,4-dichlorophenyl)-4-methyl-1H-pyrazol-3-yl)-N-(furan-3-ylmethyl)acrylamide; (E)-4-(5-(4-chlorophenyl)-1-(2,4-dichlorophenyl)-4-methyl-1H-pyrazol-3-yl)-1-(piperidin-4-yl)acrylamide; 2-(5-(4-chlorophenyl)-1-(2,4-dichlorophenyl)-4-methyl-1H-pyrazol-3-yl)-2-oxo-N-phenylacetamide; 2-(5-(4-chlorophenyl)-1-(2,4-dichlorophenyl)-4-methyl-1H-pyrazol-3-yl)-N-methyl-2-oxo-N-phenylacetamide; N-(3-chlorobenzyl)-2-(5-(4-chlorophenyl)-1-(2,4-dichlorophenyl)-4-methyl-1H-pyrazol-3-yl)-2-oxoacetamide; N-(3-chlorophenethyl)-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-N-cyclopentyl-2-oxoacetamide; 2-(5-(4-chlorophenyl)-1-(2,4-dichlorophenyl)-4-methyl-1H-pyrazol-3-yl)-N-cyclohexyl-2-oxoacetamide; 2-(5-(4-chlorophenyl)-1-(2,4-dichlorophenyl)-4-methyl-1H-pyrazol-3-yl)-N-cycloheptyl-2-oxoacetamide; 1-(5-(4-chlorophenyl)-1-(2,4-dichlorophenyl)-4-methyl-1H-pyrazol-3-yl)-2-(pyrrolidin-1-yl)ethane-1,2-dione; 1-(5-(4-chlorophenyl)-1-(2,4-dichlorophenyl)-4-methyl-1H-pyrazol-3-yl)-2-(piperidin-1-yl)ethane-1,2-dione; 1-(azepan-1-yl)-2-(5-(4-chlorophenyl)-1-(2,4-dichlorophenyl)-4-methyl-1H-pyrazol-3-yl)ethane-1,2-dione; 2-(5-(4-chlorophenyl)-1-(2,4-dichlorophenyl)-4-methyl-1H-pyrazol-3-yl)-2-oxo-N-(piperidin-1-yl)acetamide; 2-(5-(4-chlorophenyl)-1-(2,4-dichlorophenyl)-4-methyl-1H-pyrazol-3-yl)-N-(cyclohexylmethyl)-2-oxoacetamide; 1-(5-(4-chlorophenyl)-1-(2,4-dichlorophenyl)-4-methyl-1H-pyrazol-3-yl)-2-(piperazin-1-yl)ethane-1,2-dione; tert-butyl 4-(2-(5-(4-chlorophenyl)-1-(2,4-dichlorophenyl)-4-methyl-1H-pyrazol-3-yl)-2-oxoacetyl)piperazine-1-carboxylate; 1-(5-(4-chlorophenyl)-1-(2,4-dichlorophenyl)-4-methyl-1H-pyrazol-3-yl)-2-(4-methylpiperazin-1-yl)ethane-1,2-dione; 1-(5-(4-chlorophenyl)-1-(2,4-dichlorophenyl)-4-methyl-1H-pyrazol-3-yl)-2-morpholinoethane-1,2-dione; 1-(5-(4-chlorophenyl)-1-(2,4-dichlorophenyl)-4-methyl-1H-pyrazol-3-yl)-2-thiomorpholinoethane-1,2-dione; 2-(5-(4-chlorophenyl)-1-(2,4-dichlorophenyl)-4-methyl-1H-pyrazol-3-yl)-N-adamantyl-2-oxoacetamide; 2-(5-(4-chlorophenyl)-1-(2,4-dichlorophenyl)-4-methyl-1H-pyrazol-3-yl)-N,N-dicyclohexyl-2-oxoacetamide; 2-(5-(4-chlorophenyl)-1-(2,4-dichlorophenyl)-4-methyl-1H-pyrazol-3-yl)-N,N-dihexyl-2-oxoacetamide; 2-(5-(4-chlorophenyl)-1-(2,4-dichlorophenyl)-4-methyl-1H-pyrazol-3-yl)-N-ethyl-2-oxoacetamide; 2-(5-(4-chlorophenyl)-1-(2,4-dichlorophenyl)-4-methyl-1H-pyrazol-3-yl)-2-oxo-N-propylacetamide; 2-(5-(4-chlorophenyl)-1-(2,4-dichlorophenyl)-4-methyl-1H-pyrazol-3-yl)-N-isopropyl-2-oxoacetamide; N-butyl-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-N-isobutyl-2-oxoacetamide; N-tert-butyl-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-2-oxo-N-pentylacetamide; 2-(5-(4-chlorophenyl)-1-(2,4-dichlorophenyl)-4-methyl-1H-pyrazol-3-yl)-N-hexyl-2-oxoacetamide; 2-(5-(4-chlorophenyl)-1-(2,4-dichlorophenyl)-4-methyl-1H-pyrazol-3-yl)-N-heptyl-2-oxoacetamide; 2-(5-(4-chlorophenyl)-1-(2,4-dichlorophenyl)-4-methyl-1H-pyrazol-3-yl)-N-octyl-2-oxoacetamide; and 2-(5-(4-chlorophenyl)-1-(2,4-dichlorophenyl)-4-methyl-1H-pyrazol-3-yl)-N-(2-hydroxyethyl)-2-oxoacetamide, but is not limited thereto.

The lH-pyrazole-3-amide compound may be (E)-N-(3-chlorobenzyl)-3-(5-(4-chlorophenyl)-1-(2,4-dichlorophenyl)-4-methyl-1H-pyrazol-3-yl)acrylamide or 2-(5-(4-chlorophenyl)-1-(2,4-dichlorophenyl)-4-methyl-1H-pyrazol-3-yl)-N-(2-hydroxyethyl)-2-oxoacetamide.

The inflammatory skin disease may be selected from the group consisting of atopic dermatitis, allergic dermatitis, psoriasis, seborrheic dermatitis, contact dermatitis, lupus erythematosus, and papular urticaria, but is not limited thereto.

The health functional food may contain various nutritional supplements, vitamins, minerals (electrolytes), flavor agents such as synthetic flavor agents and natural flavor agents, coloring agents and thickening agents (cheese, chocolate, etc.), pectic acid and salts thereof, alginate and salts thereof, organic acids, protective colloidal thickeners, pH regulators, stabilizers, preservatives, glycerin, alcohol, and carbonating agents used in carbonated beverages. In addition, it may contain pulp for the manufacture of natural fruit juices, synthetic fruit juices, and vegetable drinks. These ingredients may be used independently or in combination. In addition, the health functional food composition may be in any form of meat, sausage, bread, chocolate, candy, snacks, confectionery, pizza, instant noodles, chewing gum, ice cream, soups, beverages, teas, functional waters, drinks, alcohol, and vitamin complexes. In addition, the health functional food may further include food additives, and the suitability as the "food additive" is determined by the standards and criteria related to corresponding items according to the general rules and general test methods of Korean Food Additives Codex approved by the Ministry of Food and Drug Safety, unless otherwise stipulated. The items listed in the "Korean Food Additives Codex" may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents. Here, the active ingredient added to the food in the process of manufacturing the health functional food may be appropriately adjusted as needed, and preferably it may be added to be included in an amount of 1 part by weight to 90 parts by weight per 100 parts by weight of food.

### Modes for Carrying Out the Invention

Hereinafter, the present disclosure will be described in more detail through examples to help understanding of the present disclosure. However, examples below are merely intended to illustrate the present disclosure, and the scope of the present disclosure is not limited to the following examples. Examples of the present disclosure are provided to more completely explain the present disclosure to those skilled in the art.

<Synthesis Example 1> Synthesis of 1H-pyrazole-3-amide compound derivative

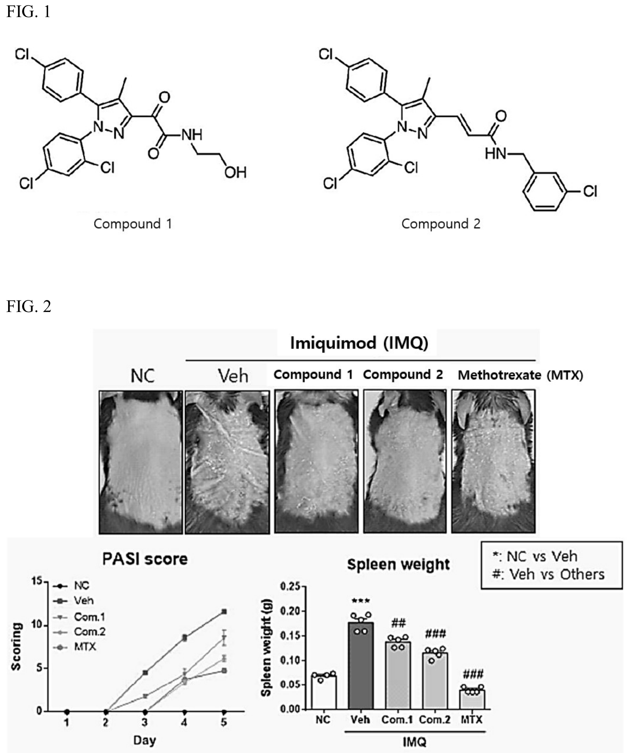

The 1H-pyrazole-3-amide compound derivative used in the present disclosure was synthesized and manufactured by performing a method disclosed in Korean Patent No. 10-1070176 with reference to this patent. Among the above compounds, an experiment was conducted to identify a therapeutic effect on inflammatory skin diseases using 2-(5-(4-chlorophenyl)-1-(2,4-dichlorophenyl)-4-methyl-1H-pyrazol-3-yl)-N-(2-hydroxyethyl)-2-oxoacetamide (named Compound 1) and (E)-N-(3-chlorobenzyl)-3-(5-(4-chlorophenyl)-1-(2,4-dichlorophenyl)-4-methyl-1H-pyrazol-3-yl)acrylamide (named Compound 2). At this time, the structural formulas of the Compound 1 and Compound 2 were as shown in FIG. 1.

### <Example 1> Identification of therapeutic effect on psoriasis

### (1) Preparation of psoriasis animal model and measurement of PSAI score and spleen weight

The hair on the backs of 7-week-old mice was removed, and then 62.5 mg of Aldara cream was applied daily for 4 days. 1% DMSO and 5% kolliphor in PBS were mixed to be injected into the vehicle intraperitoneally daily. 20 mg/kg of Compound 1, 10 mg/kg of Compound 2, and 10mg/kg of MTx were prepared in body weight x 10 mg/kg, which was injected intraperitoneally daily. After injection, the mice were left for 30 minutes for sufficient suppression of cannabinoid receptor 1, applied with Aldara cream, and then sacrificed on day 5 from day 1 at which the Aldara cream was first applied, and skin tissues were made into paraffin blocks. These were evenly cut into 4 µm thick pieces, and immunochemical staining was performed.

As a result, according to FIG. 2, it was found that the model injected with Compound 1 or Compound 2 had a lower PASI score and a decrease in the spleen weight compared to a vehicle group.

### (2) Determination of skin thickness in psoriasis mouse model

The slides were placed in a 60°C oven for 1 hour, rinsed with xylene solution for 5 minutes, 100% EtOH for 5 minutes, 70% EtOH for 5 minutes, distilled water, and PBS, and then immersed in hematoxylin for 10 seconds and eosin for 3 seconds. Afterwards, they were rinsed with 70% EtOH and 100% EtOH, immersed in xylene for 20 seconds, sprayed with mounting solution, and covered with a cover glass. Each section of the skin tissue was photographed using a Leica microscope and then quantified by setting three sections per tissue as the average.

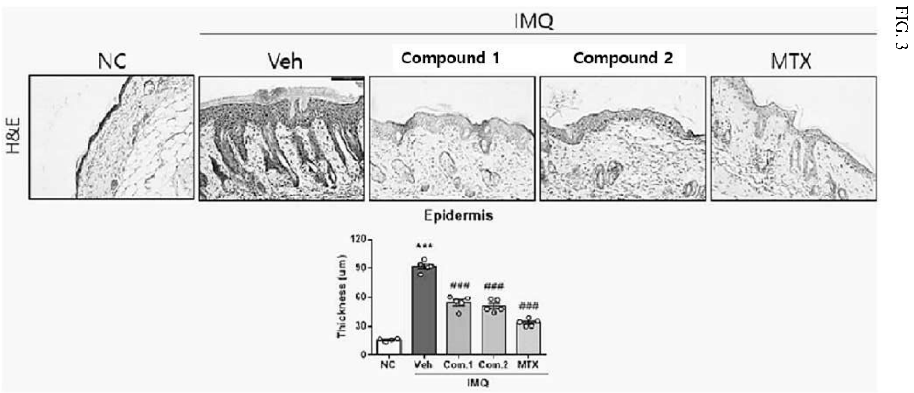

As a result, according to FIG. 3, compared to the vehicle group in which the epidermis and dermis thickened as psoriasis was induced, it was found that the thickness of the epidermis was significantly reduced by intraperitoneal injection of Compound 1 or Compound 2 to inhibit CB1R.

### (3) Identification of TNF-α and IL-1β reduction effects in psoriasis mouse model

For immunohistochemical staining, the slides were placed in a 60°C oven for 1 hour, rinsed with xylene solution for 5 minutes, 100% EtOH for 5 minutes, 70% EtOH for 5 minutes, distilled water, and PBS, then immersed in citrate buffer, placed in a 100°C oven for 30 minutes, and slowly cooled at room temperature for 45 minutes. After rinsing with distilled water, the slides were soaked in PBS for 5 minutes, sprayed with 0.25% TritonX-100, and left for 10 minutes to disrupt the cell membrane and allow the antibodies to penetrate. After rinsing with 0.1% Tween 20 in PBS (hereinafter referred to as PBST), hydrogen peroxide block buffer (abcam59717) was sprayed and left for 10 minutes. Afterwards, after rinsing with PBST, the slides were sprayed with 150 µl of normal blocking serum (VectastatinABC kit, PK-4001) mixed in 10 ml of PBS. The primary antibody was mixed with R.T.U animal free block and diluent (SP-5035) in 1:500, spread on the slides, and reacted in a refrigerator at 4°C for 14 hours. After rinsing with PBST, the secondary antibody was applied for 1 hour and then HRP (VectastatinABC reagent) for 30 minutes. DAB substrate was mixed in PBS and spread on the slides to carry out a reaction for the same amount of time, counterstaining was performed with hematoxylin followed by rinsing with 70% EtOH and 100% EtOH, and then it was immersed in xylene for 20 seconds, sprayed with mounting solution, and covered with a cover glass.

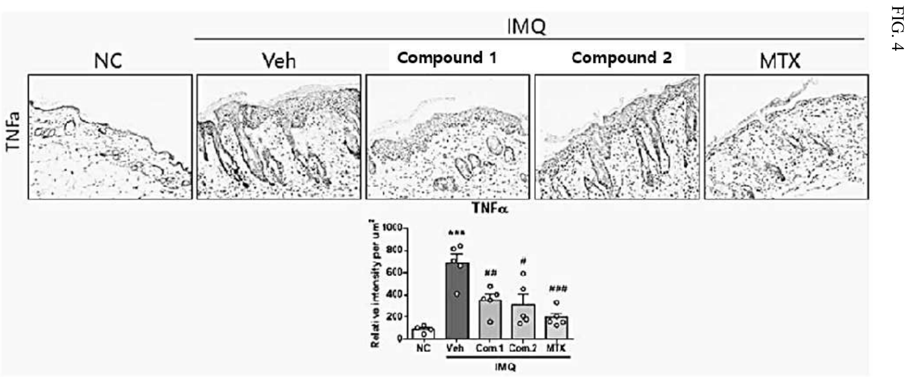

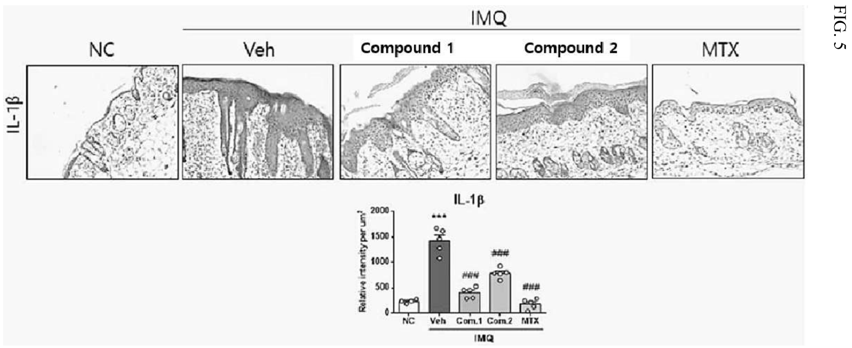

The signals of TNF-α and IL-1β, stained brown, were quantified through immunohistochemical staining. As a result, according to FIG. 4, it was found that the expression level of TNF-α, an inflammatory factor that increases in the epidermis as psoriasis is induced, was significantly reduced compared to the vehicle group when cannabinoid receptor 1 was inhibited by intraperitoneal injection of Compound 1 or Compound 2, and according to FIG. 5, it was found that the expression level of IL-1β, an inflammatory factor that increases in the epidermis as psoriasis is induced, was significantly reduced compared to the vehicle group when cannabinoid receptor 1 was inhibited by intraperitoneal injection of Compound 1 or Compound 2. Here, it was found that the inhibitory effect of Compound 1 was higher than that of Compound 2.

### <Example 2> Identification of therapeutic effect on contact dermatitis

### (1) Preparation of animal model with contact dermatitis and identification of therapeutic effect on dermatitis

After removing hair from the ears of 7-week-old mice, on the day after hair removal (day 1), oxazolone (4-ethoxymethylene-2-phenyl-2-oxazolin-5-one, cat#E0753-1G, sigma-Aldrich) was mixed in acetone and olive oil in a concentration of 2%, which was then applied to the inner and outer sides of the ears by 5µl each. 1% DMSO and 5% kolliphor in PBS were mixed to be injected into the vehicle intraperitoneally daily. Intraperitoneal injection was conducted daily by 20 mg/kg for Compound 2 and 10 mg/kg for Compound 1 with a 30 interval for sufficient suppression of cannabinoid receptor 1 after the injection, and then the size of the ears was measured, followed by application of oxazolone (on days 1 and 6). On day 6, 1% oxazolone was prepared and applied to the inner and outer side of the ears by 5 µl each, and on day 7, the mice were sacrificed, with the ears formed into paraffin blocks and then cut into 4 µm thick pieces to be placed on slides.

The 2% oxazolone used on day 1 was finally prepared in a volume of 800 µl for 20 mice, 16 mg of oxazolone was first dissolved in 160 µl of olive oil, then 640 µl of acetone was additionally mixed, and 10 µl of the mixture was applied on both ears, 5 µl for the front and 5 µl for the back per ear. The 1% oxazolone used on day 6 was finally prepared in a volume of 800 µl for 20 mice, 8 mg of oxazolone was first dissolved in 160 µl of olive oil, then 640 µl of acetone was additionally mixed, and 10 µl of the mixture was applied on both ears, 5 µl for the front and 5 µl for the back per ear.

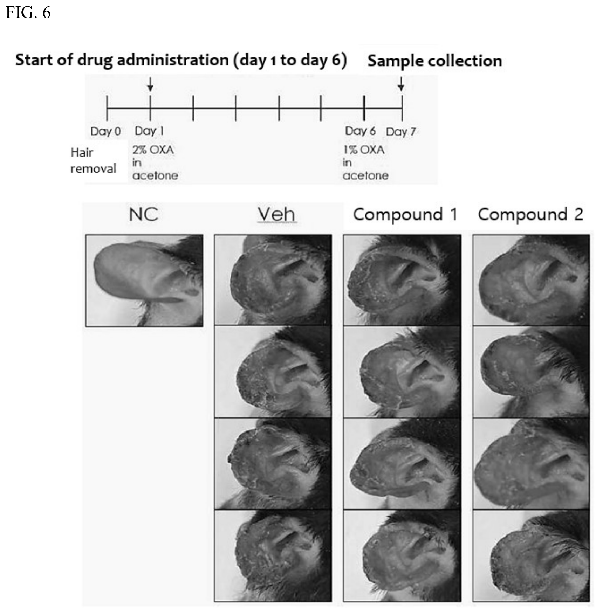

As a result, according to FIG. 6, it was found that the symptoms of contact dermatitis were reduced in the model to which Compound 1 or Compound 2 was applied compared to the vehicle group.

### (2) Therapeutic effect on dermatitis in contact dermatitis mouse model

The slides were placed in a 60°C oven for 1 hour, rinsed with xylene solution for 5 minutes, 100% EtOH for 5 minutes, 70% EtOH for 5 minutes, distilled water, and PBS, and then immersed in hematoxylin for 10 seconds and eosin for 3 seconds. Afterwards, they were rinsed with 70% EtOH and 100% EtOH, immersed in xylene for 20 seconds, sprayed with mounting solution, and covered with a cover glass. Each section of skin tissue was photographed using a Leica microscope and then quantified by setting three sections per tissue as the average.

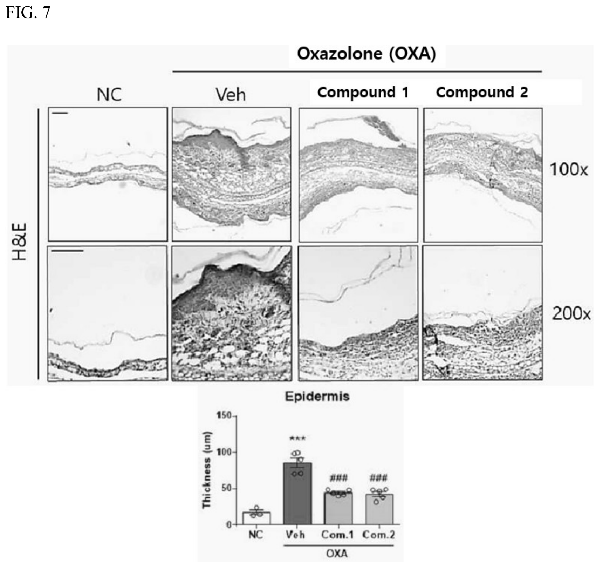

As a result, according to FIG. 7, compared to the vehicle group in which the epidermis thickened as contact dermatitis was induced, it was found that the thickness of the ear skin was significantly reduced by intraperitoneal injection of Compound 1 or Compound 2 to inhibit CB1R.

### (3) Identification of IL-1β, IL-17A, and ki67 reduction effects in contact dermatitis mouse model

The signals of IL-1β, IL-17A, and ki67, stained brown, were quantified through immunohistochemical staining.

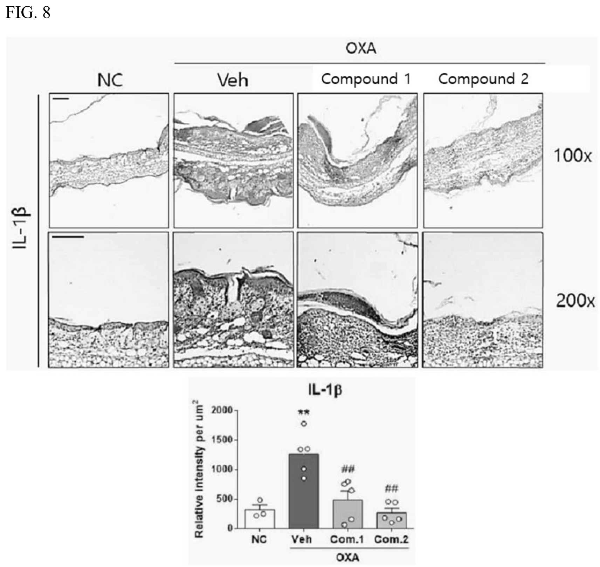

As a result, according to FIG. 8, it was found that the expression level of IL-1β, an inflammatory factor that increases in the epidermis as contact dermatitis is induced, was significantly reduced compared to the vehicle group when cannabinoid receptors were inhibited by intraperitoneal injection of Compound 1 or Compound 2.

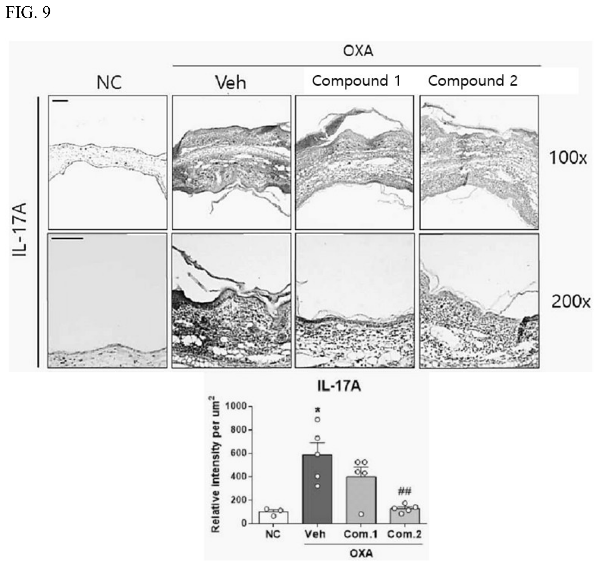

In addition, according to FIG. 9, it was found that the expression level of IL-17A, an inflammatory factor that increases as contact dermatitis is induced, was significantly reduced compared to the vehicle group when cannabinoid receptors were inhibited by intraperitoneal injection of Compound 1 or Compound 2.

The foregoing description of the present disclosure is for illustrative purposes only, and those skilled in the art to which the present disclosure pertains will appreciate that the present disclosure may be easily modified into other concrete forms without altering the technical spirit or essential characteristics of the present disclosure. Therefore, it should be understood that the examples described above are illustrative in all respects but not limiting.

The scope of the present disclosure is indicated by the appended claims, and all changes or modified forms derived from the meaning and scope of the claims and equivalent concepts thereof should be construed as being included in the scope of the present disclosure.

## Claims

1. A pharmaceutical composition for preventing or treating an inflammatory skin disease, comprising a 1H-pyrazole-3-amide compound represented by the following Chemical Formula 1 or a pharmaceutically acceptable salt thereof as an active ingredient; 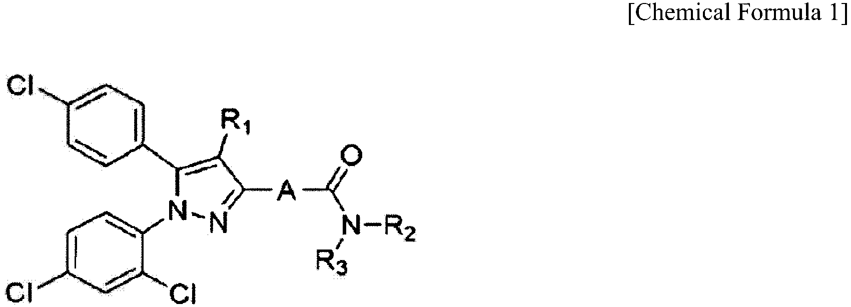 wherein, in the Chemical Formula 1,
A is 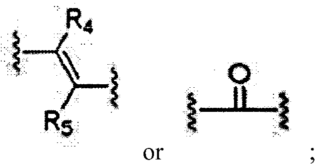
R₁ is hydrogen, (C1-C10)alkyl, (C3-C7)cycloalkyl, (C6-C20)aryl, or (C6-C20)ar(C1-C 10)alkyl;
R₂ and R₃ are independently hydrogen, (C1-C10)alkyl, (C6-C20)aryl, (C6-C20)ar(C1-C10)alkyl, (C3-C7)cycloalkyl, (C3-C7)heterocycloalkyl comprising one or more heteroatoms selected from N, O, and S, adamantyl, piperidino, or pyrrolidino or R₂ and R₃ may be connected to (C3-C7)alkylene to form a ring, and one or more carbon atoms in the alkylene may be substituted with NR₆, O, or S;
R₄ and R₅ are each independently hydrogen, (C1-C10)alkyl, (C3-C7)cycloalkyl, or (C6-C20)aryl;
R₆ is hydrogen, (C1-C10)alkyl, or (C1-C10)alkoxycarbonyl; and
the alkyl, aryl, aralkyl, cycloalkyl, adamantly, or heterocycloalkyl in the R₁, R₂, and R₃ may be further substituted with one or more substituents selected from halogen, (C1-C10)alkyl, halo(C1-C10)alkyl, (C1-C10)alkoxy, mono or di-(C1-C10)alkylamino, mono or di-(C6-C20)arylamino, (C3-C7)cycloalkyl, (C2-C20)heteroaryl, hydroxy, (C3-C7)heterocycloalkyl comprising one or more heteroatoms selected from N, O, and S, pyrrolidino, or piperidino.

2. The pharmaceutical composition of claim 1, wherein, in the Chemical Formula 1,
R₁ is (C1-C10)alkyl, R₂ and R₃ are each independently hydrogen, (C1-C10)alkyl, (C6-C20)aryl, (C6-C20)ar(C1-C10)alkyl, (C3-C7)cycloalkyl, piperidino, adamantyl, or piperidinyl or R₂ and R₃ may be connected to (C4-C6)alkylene to form a ring, and one or more carbon atoms in the alkylene may be substituted with NR₆, O, or S;
R₄ and R₅ are hydrogen;
R₆ is hydrogen, (C1-C10)alkyl, or (C1-C10)alkoxycarbonyl; and
the alkyl, aryl, or aralkyl in the R₂ and R₃ may be further substituted with one or more substituents selected from halogen, (C1-C10)alkyl, halo(C1-C10)alkyl, (C1-C10)alkoxy, mono or di-(C1-C10)alkylamino, (C3-C7)cycloalkyl, (C2-C20)heteroaryl, or hydroxy.

3. The pharmaceutical composition of claim 1, wherein the 1H-pyrazol-3-amide compound is selected from the group consisting of (E)-N-(4-chlorophenyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-phenylacrylamide; (E)-N-(3-chlorobenzyl)-3-(5-(4-chlorophenyl)-1-(2,4-dichlorophenyl)-4-methyl-1H-pyrazol-3-yl)acrylamide; (E)-N-(2-chlorobenzyl)-3-(5-(4-chlorophenyl)-1-(2,4-dichlorophenyl)-4-methyl-1H-pyrazol-3-yl)acrylamide; (E)-N-benzyl-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(4-methoxybenzyl)acrylamide; (E)-N-(4-chlorobenzyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(4-fluorobenzyl)acrylamide; (E)-3-(5-(4-chlorophenyl)-1-(2,4-dichlorophenyl)-4-methyl-1H-pyrazol-3-yl)-N-(3-fluorobenzyl)acrylamide; (E)-3-(5-(4-chlorophenyl)-1-(2,4-dichlorophenyl)-4-methyl-1H-pyrazol-3-yl)-N-isopropylacrylamide; (E)-3-(5-(4-chlorophenyl)-1-(2,4-dichlorophenyl)-4-methyl-1H-pyrazol-3-yl)-N-(3-chloropropyl)acrylamide; (E)-3-(5-(4-chlorophenyl)-1-(2,4-dichlorophenyl)-4-methyl-1H-pyrazol-3-yl)-N-hexylacrylamide; (E)-3-(5-(4-chlorophenyl)-1-(2,4-dichlorophenyl)-4-methyl-1H-pyrazol-3-yl)-N-(cyclohexylmethyl)acrylamide; (E)-3-(5-(4-chlorophenyl)-1-(2,4-dichlorophenyl)-4-methyl-1H-pyrazol-3-yl)-N-(3-(trifluoromethyl)benzyl)acrylamide; (E)-3-(5-(4-chlorophenyl)-1-(2,4-dichlorophenyl)-4-methyl-1H-pyrazol-3-yl)-N-(4-trifluoromethyl)benzyl)acrylamide; (E)-3-(5-(4-chlorophenyl)-1-(2,4-dichlorophenyl)-4-methyl-1H-pyrazol-3-yl)-N-(4-(dimethylamino)benzyl)acrylamide; (E)-N-(3-chlorophenethyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(piperidin-1-yl)acrylamide; (E)-3-(5-(4-chlorophenyl)-1-(2,4-dichlorophenyl)-4-methyl-1H-pyrazol-3-yl)-N-(furan-3-ylmethyl)acrylamide; (E)-4-(5-(4-chlorophenyl)-1-(2,4-dichlorophenyl)-4-methyl-1H-pyrazol-3-yl)-1-(piperidin-4-yl)acrylamide; 2-(5-(4-chlorophenyl)-1-(2,4-dichlorophenyl)-4-methyl-1H-pyrazol-3-yl)-2-oxo-N-phenylacetamide; 2-(5-(4-chlorophenyl)-1-(2,4-dichlorophenyl)-4-methyl-1H-pyrazol-3-yl)-N-methyl-2-oxo-N-phenylacetamide; N-(3-chlorobenzyl)-2-(5-(4-chlorophenyl)-1-(2,4-dichlorophenyl)-4-methyl-1H-pyrazol-3-yl)-2-oxoacetamide; N-(3-chlorophenethyl)-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-N-cyclopentyl-2-oxoacetamide; 2-(5-(4-chlorophenyl)-1-(2,4-dichlorophenyl)-4-methyl-1H-pyrazol-3-yl)-N-cyclohexyl-2-oxoacetamide; 2-(5-(4-chlorophenyl)-1-(2,4-dichlorophenyl)-4-methyl-1H-pyrazol-3-yl)-N-cycloheptyl-2-oxoacetamide; 1-(5-(4-chlorophenyl)-1-(2,4-dichlorophenyl)-4-methyl-1H-pyrazol-3-yl)-2-(pyrrolidin-1-yl)ethane-1,2-dione; 1-(5-(4-chlorophenyl)-1-(2,4-dichlorophenyl)-4-methyl-1H-pyrazol-3-yl)-2-(piperidin-1-yl)ethane-1,2-dione; 1-(azepan-1-yl)-2-(5-(4-chlorophenyl)-1-(2,4-dichlorophenyl)-4-methyl-1H-pyrazol-3-yl)ethane-1,2-dione; 2-(5-(4-chlorophenyl)-1-(2,4-dichlorophenyl)-4-methyl-1H-pyrazol-3-yl)-2-oxo-N-(piperidin-1-yl)acetamide; 2-(5-(4-chlorophenyl)-1-(2,4-dichlorophenyl)-4-methyl-1H-pyrazol-3-yl)-N-(cyclohexylmethyl)-2-oxoacetamide; 1-(5-(4-chlorophenyl)-1-(2,4-dichlorophenyl)-4-methyl-1H-pyrazol-3-yl)-2-(piperazin-1-yl)ethane-1,2-dione; tert-butyl 4-(2-(5-(4-chlorophenyl)-1-(2,4-dichlorophenyl)-4-methyl-1H-pyrazol-3-yl)-2-oxoacetyl)piperazine-1-carboxylate; 1-(5-(4-chlorophenyl)-1-(2,4-dichlorophenyl)-4-methyl-1H-pyrazol-3-yl)-2-(4-methylpiperazin-1-yl)ethane-1,2-dione; 1-(5-(4-chlorophenyl)-1-(2,4-dichlorophenyl)-4-methyl-1H-pyrazol-3-yl)-2-morpholinoethane-1,2-dione; 1-(5-(4-chlorophenyl)-1-(2,4-dichlorophenyl)-4-methyl-1H-pyrazol-3-yl)-2-thiomorpholinoethane-1,2-dione; 2-(5-(4-chlorophenyl)-1-(2,4-dichlorophenyl)-4-methyl-1H-pyrazol-3-yl)-N-adamantyl-2-oxoacetamide; 2-(5-(4-chlorophenyl)-1-(2,4-dichlorophenyl)-4-methyl-1H-pyrazol-3-yl)-N,N-dicyclohexyl-2-oxoacetamide; 2-(5-(4-chlorophenyl)-1-(2,4-dichlorophenyl)-4-methyl-1H-pyrazol-3-yl)-N,N-dihexyl-2-oxoacetamide; 2-(5-(4-chlorophenyl)-1-(2,4-dichlorophenyl)-4-methyl-1H-pyrazol-3-yl)-N-ethyl-2-oxoacetamide; 2-(5-(4-chlorophenyl)-1-(2,4-dichlorophenyl)-4-methyl-1H-pyrazol-3-yl)-2-oxo-N-propylacetamide; 2-(5-(4-chlorophenyl)-1-(2,4-dichlorophenyl)-4-methyl-1H-pyrazol-3-yl)-N-isopropyl-2-oxoacetamide; N-butyl-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-N-isobutyl-2-oxoacetamide; N-tert-butyl-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-2-oxo-N-pentylacetamide; 2-(5-(4-chlorophenyl)-1-(2,4-dichlorophenyl)-4-methyl-1H-pyrazol-3-yl)-N-hexyl-2-oxoacetamide; 2-(5-(4-chlorophenyl)-1-(2,4-dichlorophenyl)-4-methyl-1H-pyrazol-3-yl)-N-heptyl-2-oxoacetamide; 2-(5-(4-chlorophenyl)-1-(2,4-dichlorophenyl)-4-methyl-1H-pyrazol-3-yl)-N-octyl-2-oxoacetamide; and 2-(5-(4-chlorophenyl)-1-(2,4-dichlorophenyl)-4-methyl-1H-pyrazol-3-yl)-N-(2-hydroxyethyl)-2-oxoacetamide.

4. The pharmaceutical composition of claim 1, wherein the 1H-pyrazole-3-amide compound is (E)-N-(3-chlorobenzyl)-3-(5-(4-chlorophenyl)-1-(2,4-dichlorophenyl)-4-methyl-1H-pyrazol-3-yl)acrylamide or 2-(5-(4-chlorophenyl)-1-(2,4-dichlorophenyl)-4-methyl-1H-pyrazol-3-yl)-N-(2-hydroxyethyl)-2-oxoacetamide.

5. The pharmaceutical composition of claim 1, wherein the inflammatory skin disease is selected from the group consisting of atopic dermatitis, allergic dermatitis, psoriasis, seborrheic dermatitis, contact dermatitis, lupus erythematosus, and papular urticaria.

6. A health functional food composition for preventing or ameliorating an inflammatory skin disease, comprising a 1H-pyrazole-3-amide compound represented by the following Chemical Formula 1 or a pharmaceutically acceptable salt thereof as an active ingredient; 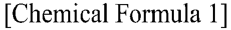 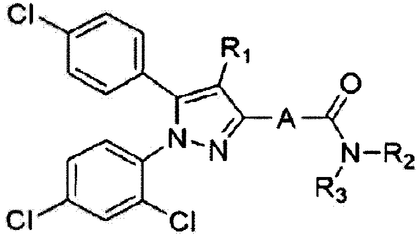 wherein, in the Chemical Formula 1,
A is 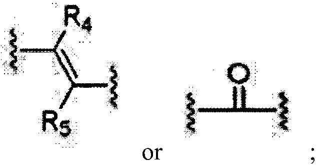
R₁ is hydrogen, (C1-C10)alkyl, (C3-C7)cycloalkyl, (C6-C20)aryl, or (C6-C20)ar(C1-C 10)alkyl;
R₂ and R₃ are independently hydrogen, (C1-C10)alkyl, (C6-C20)aryl, (C6-C20)ar(C1-C10)alkyl, (C3-C7)cycloalkyl, (C3-C7)heterocycloalkyl comprising one or more heteroatoms selected from N, O, and S, adamantyl, piperidino, or pyrrolidino or R₂ and R₃ may be connected to (C3-C7)alkylene to form a ring, and one or more carbon atoms in the alkylene may be substituted with NR₆, O, or S;
R₄ and R₅ are each independently hydrogen, (C1-C10)alkyl, (C3-C7)cycloalkyl, or (C6-C20)aryl; R₆ is hydrogen, (C1-C10)alkyl, or (C1-C10)alkoxycarbonyl; and
the alkyl, aryl, aralkyl, cycloalkyl, adamantly, or heterocycloalkyl in the R₁, R₂, and R₃ may be further substituted with one or more substituents selected from halogen, (C1-C10)alkyl, halo(C1-C10)alkyl, (C1-C10)alkoxy, mono or di-(C1-C10)alkylamino, mono or di-(C6-C20)arylamino, (C3-C7)cycloalkyl, (C2-C20)heteroaryl, hydroxy, (C3-C7)heterocycloalkyl comprising one or more heteroatoms selected from N, O, and S, pyrrolidino, or piperidino.

7. The health functional food composition of claim 6, wherein, in the Chemical Formula 1,
R₁ is (C1-C10)alkyl, R₂ and R₃ are each independently hydrogen, (C1-C10)alkyl, (C6-C20)aryl, (C6-C20)ar(C1-C10)alkyl, (C3-C7)cycloalkyl, piperidino, adamantyl, or piperidinyl or R₂ and R₃ may be connected to (C4-C6)alkylene to form a ring, and one or more carbon atoms in the alkylene may be substituted with NR₆, O, or S;
R₄ and R₅ are hydrogen;
R₆ is hydrogen, (C1-C10)alkyl, or (C1-C10)alkoxycarbonyl; and
the alkyl, aryl, or aralkyl in the R₂ and R₃ may be further substituted with one or more substituents selected from halogen, (C1-C10)alkyl, halo(C1-C10)alkyl, (C1-C10)alkoxy, mono or di-(C1-C10)alkylamino, (C3-C7)cycloalkyl, (C2-C20)heteroaryl, or hydroxy.

8. The health functional food composition of claim 6, wherein the 1H-pyrazol-3-amide compound is selected from the group consisting of (E)-N-(4-chlorophenyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-phenylacrylamide; (E)-N-(3-chlorobenzyl)-3-(5-(4-chlorophenyl)-1-(2,4-dichlorophenyl)-4-methyl-1H-pyrazol-3-yl)acrylamide; (E)-N-(2-chlorobenzyl)-3-(5-(4-chlorophenyl)-1-(2,4-dichlorophenyl)-4-methyl-1H-pyrazol-3-yl)acrylamide; (E)-N-benzyl-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(4-methoxybenzyl)acrylamide; (E)-N-(4-chlorobenzyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(4-fluorobenzyl)acrylamide; (E)-3-(5-(4-chlorophenyl)-1-(2,4-dichlorophenyl)-4-methyl-1H-pyrazol-3-yl)-N-(3-fluorobenzyl)acrylamide; (E)-3-(5-(4-chlorophenyl)-1-(2,4-dichlorophenyl)-4-methyl-1H-pyrazol-3-yl)-N-isopropylacrylamide; (E)-3-(5-(4-chlorophenyl)-1-(2,4-dichlorophenyl)-4-methyl-1H-pyrazol-3-yl)-N-(3-chloropropyl)acrylamide; (E)-3-(5-(4-chlorophenyl)-1-(2,4-dichlorophenyl)-4-methyl-1H-pyrazol-3-yl)-N-hexylacrylamide; (E)-3-(5-(4-chlorophenyl)-1-(2,4-dichlorophenyl)-4-methyl-1H-pyrazol-3-yl)-N-(cyclohexylmethyl)acrylamide; (E)-3-(5-(4-chlorophenyl)-1-(2,4-dichlorophenyl)-4-methyl-1H-pyrazol-3-yl)-N-(3-(trifluoromethyl)benzyl)acrylamide; (E)-3-(5-(4-chlorophenyl)-1-(2,4-dichlorophenyl)-4-methyl-1H-pyrazol-3-yl)-N-(4-trifluoromethyl)benzyl)acrylamide; (E)-3-(5-(4-chlorophenyl)-1-(2,4-dichlorophenyl)-4-methyl-1H-pyrazol-3-yl)-N-(4-(dimethylamino)benzyl)acrylamide; (E)-N-(3-chlorophenethyl)-3-(5-(4-chlorophenyl)-1-(2,4-dichlorophenyl)-4-methyl-1H-pyrazol-3-yl)acrylamide; (E)-3-(5-(4-chlorophenyl)-1-(2,4-dichlorophenyl)-4-methyl-1H-pyrazol-3-yl)-N-(piperidin-1-yl)acrylamide; (E)-3-(5-(4-chlorophenyl)-1-(2,4-dichlorophenyl)-4-methyl-1H-pyrazol-3-yl)-N-(furan-3-ylmethyl)acrylamide; (E)-4-(5-(4-chlorophenyl)-1-(2,4-dichlorophenyl)-4-methyl-1H-pyrazol-3-yl)-1-(piperidin-4-yl)acrylamide; 2-(5-(4-chlorophenyl)-1-(2,4-dichlorophenyl)-4-methyl-1H-pyrazol-3-yl)-2-oxo-N-phenylacetamide; 2-(5-(4-chlorophenyl)-1-(2,4-dichlorophenyl)-4-methyl-1H-pyrazol-3-yl)-N-methyl-2-oxo-N-phenylacetamide; N-(3-chlorobenzyl)-2-(5-(4-chlorophenyl)-1-(2,4-dichlorophenyl)-4-methyl-1H-pyrazol-3-yl)-2-oxoacetamide; N-(3-chlorophenethyl)-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-N-cyclopentyl-2-oxoacetamide; 2-(5-(4-chlorophenyl)-1-(2,4-dichlorophenyl)-4-methyl-1H-pyrazol-3-yl)-N-cyclohexyl-2-oxoacetamide; 2-(5-(4-chlorophenyl)-1-(2,4-dichlorophenyl)-4-methyl-1H-pyrazol-3-yl)-N-cycloheptyl-2-oxoacetamide; 1-(5-(4-chlorophenyl)-1-(2,4-dichlorophenyl)-4-methyl-1H-pyrazol-3-yl)-2-(pyrrolidin-1-yl)ethane-1,2-dione; 1-(5-(4-chlorophenyl)-1-(2,4-dichlorophenyl)-4-methyl-1H-pyrazol-3-yl)-2-(piperidin-1-yl)ethane-1,2-dione; 1-(azepan-1-yl)-2-(5-(4-chlorophenyl)-1-(2,4-dichlorophenyl)-4-methyl-1H-pyrazol-3-yl)ethane-1,2-dione; 2-(5-(4-chlorophenyl)-1-(2,4-dichlorophenyl)-4-methyl-1H-pyrazol-3-yl)-2-oxo-N-(piperidin-1-yl)acetamide; 2-(5-(4-chlorophenyl)-1-(2,4-dichlorophenyl)-4-methyl-1H-pyrazol-3-yl)-N-(cyclohexylmethyl)-2-oxoacetamide; 1-(5-(4-chlorophenyl)-1-(2,4-dichlorophenyl)-4-methyl-1H-pyrazol-3-yl)-2-(piperazin-1-yl)ethane-1,2-dione; tert-butyl 4-(2-(5-(4-chlorophenyl)-1-(2,4-dichlorophenyl)-4-methyl-1H-pyrazol-3-yl)-2-oxoacetyl)piperazine-1-carboxylate; 1-(5-(4-chlorophenyl)-1-(2,4-dichlorophenyl)-4-methyl-1H-pyrazol-3-yl)-2-(4-methylpiperazin-1-yl)ethane-1,2-dione; 1-(5-(4-chlorophenyl)-1-(2,4-dichlorophenyl)-4-methyl-1H-pyrazol-3-yl)-2-morpholinoethane-1,2-dione; 1-(5-(4-chlorophenyl)-1-(2,4-dichlorophenyl)-4-methyl-1H-pyrazol-3-yl)-2-thiomorpholinoethane-1,2-dione; 2-(5-(4-chlorophenyl)-1-(2,4-dichlorophenyl)-4-methyl-1H-pyrazol-3-yl)-N-adamantyl-2-oxoacetamide; 2-(5-(4-chlorophenyl)-1-(2,4-dichlorophenyl)-4-methyl-1H-pyrazol-3-yl)-N,N-dicyclohexyl-2-oxoacetamide; 2-(5-(4-chlorophenyl)-1-(2,4-dichlorophenyl)-4-methyl-1H-pyrazol-3-yl)-N,N-dihexyl-2-oxoacetamide; 2-(5-(4-chlorophenyl)-1-(2,4-dichlorophenyl)-4-methyl-1H-pyrazol-3-yl)-N-ethyl-2-oxoacetamide; 2-(5-(4-chlorophenyl)-1-(2,4-dichlorophenyl)-4-methyl-1H-pyrazol-3-yl)-2-oxo-N-propylacetamide; 2-(5-(4-chlorophenyl)-1-(2,4-dichlorophenyl)-4-methyl-1H-pyrazol-3-yl)-N-isopropyl-2-oxoacetamide; N-butyl-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-N-isobutyl-2-oxoacetamide; N-tert-butyl-2-(5-(4-chlorophenyl)-1-(2,4-dichlorophenyl)-4-methyl-1H-pyrazol-3-yl)-2-oxoacetamide; 2-(5-(4-chlorophenyl)-1-(2,4-dichlorophenyl)-4-methyl-1H-pyrazol-3-yl)-2-oxo-N-pentylacetamide; 2-(5-(4-chlorophenyl)-1-(2,4-dichlorophenyl)-4-methyl-1H-pyrazol-3-yl)-N-hexyl-2-oxoacetamide; 2-(5-(4-chlorophenyl)-1-(2,4-dichlorophenyl)-4-methyl-1H-pyrazol-3-yl)-N-heptyl-2-oxoacetamide; 2-(5-(4-chlorophenyl)-1-(2,4-dichlorophenyl)-4-methyl-1H-pyrazol-3-yl)-N-octyl-2-oxoacetamide; and 2-(5-(4-chlorophenyl)-1-(2,4-dichlorophenyl)-4-methyl-1H-pyrazol-3-yl)-N-(2-hydroxyethyl)-2-oxoacetamide.

9. The health functional food composition of claim 6, wherein the 1H-pyrazole-3-amide compound is (E)-N-(3-chlorobenzyl)-3-(5-(4-chlorophenyl)-1-(2,4-dichlorophenyl)-4-methyl-1H-pyrazol-3-yl)acrylamide or 2-(5-(4-chlorophenyl)-1-(2,4-dichlorophenyl)-4-methyl-1H-pyrazol-3-yl)-N-(2-hydroxyethyl)-2-oxoacetamide.

10. The health functional food composition of claim 6, wherein the inflammatory skin disease is selected from the group consisting of atopic dermatitis, allergic dermatitis, psoriasis, seborrheic dermatitis, contact dermatitis, lupus erythematosus, and papular urticaria.
